# EUROPÄISCHE PATENTSCHRIFT

(11) **EP 3 237 036 B1**
(45) Veröffentlichungstag und Bekanntmachung des Hinweises auf die Patenterteilung: **17.07.2019**
(21) Anmeldenummer: 15816778.3
(22) Anmeldetag: 21.12.2015
(51) Int. Cl.: A61M 1/36, A61M 5/142, B01F 3/08, A61M 1/34

(54) **INFUSIONSLEITUNG MIT EINRICHTUNG ZUM BEGÜNSTIGEN EINER VERMISCHUNG EINER INFUSIONSLÖSUNG MIT EINEM WEITEREN FLUID**
INFUSION LINE HAVING AN APPARATUS FOR PROMOTING THE MIXING OF AN INFUSION SOLUTION WITH A FURTHER FLUID
LIGNE DE PERFUSION AVEC DISPOSITIF POUR FAVORISER LE MÉLANGE D'UNE SOLUTION DE PERFUSION AVEC UN AUTRE FLUIDE

(30) Priorität: 22.12.2014 DE 102014119445
(43) Veröffentlichungstag der Anmeldung: 01.11.2017
(73) Patentinhaber: Fresenius Medical Care Deutschland GmbH, 61352 Bad Homburg (DE)
(72) Erfinder: ETZDORF, Dirk, 61476 Kronberg (DE)
(74) Vertreter: Bobbert & Partner Patentanwälte PartmbB
(86) Internationale Anmeldenummer: PCT/EP2015/080815
(87) Internationale Veröffentlichungsnummer: WO 2016/102479

(56) Entgegenhaltungen:
- EP-A2- 0 165 519
- EP-A2- 1 666 078
- WO-A2-2007/101064
- DE-A1- 4 240 681
- US-A- 3 511 238
- US-A1- 2013 028 788

## Beschreibung

Die Erfindung betrifft eine Zugabeleitung gemäß Anspruch 1, einen extrakorporalen Blutkreislauf gemäß Anspruch 4 sowie eine Blutbehandlungsvorrichtung gemäß Anspruch 6.

Bei der extrakorporalen Blutbehandlung werden Infusionslösungen oder Medikamente meist über den extrakorporalen Blutkreislauf, d. h. das verwendete Blutschlauchsystem, infundiert. Je nach Art der Infusionslösung kann eine schnelle Mischung der Infusionslösung mit dem Blut wünschenswert sein.

So werden bei der extrakorporalen Blutbehandlung standardmäßig Infusionslösungen zur Hemmung der Blutgerinnung infundiert, um einem möglichen Verschluss des extrakorporalen Blutkreislaufs vorzubeugen.

Dazu werden hauptsächlich zwei Verfahren angewendet, die systemische und die regionale Antikoagulation. Bei der regionalen Antikoagulation wird als Antikoagulanz meist eine Citratlosung verwendet, die Calcium komplexiert und so die Gerinnung des Blutes unterdrückt. Vor Rückgabe des Bluts an den Patienten muss typischerweise zusätzliches Calcium verabreicht werden, da zu niedrige Calciumkonzentrationen Auswirkungen auf Nerven und Muskeln, die Blutgerinnung sowie Funktion von Lunge, Herz und Nieren haben. Deshalb wird bei der regionalen Antikoagulation dem Blut vor Reinfusion in den Patienten eine calciumhaltige Lösung zudosiert, durch die die physiologische Calciumkonzentration im systemischen Blut und damit gleichzeitig dessen Gerinnungsfähigkeit wiederhergestellt werden.

Die Infusion von Infusionslösungen oder Medikamenten in das Schlauchsystem des extrakorporalen Blutkreislaufs erfolgt üblicherweise über Zugabestellen in T-Form, sogenannte T-Stücke. In diesen T-Stücken herrschen aufgrund des kreisförmigen Querschnitts und der glatten Innenwandung an der Zugabestelle laminare Strömungsbedingungen vor. Zusätzlich sind die Flussraten der infundierten Lösungen im Vergleich zum Blutfluss gering.

Die weitgehend laminaren Strömungsbedingungen und die geringen Flüsse der in den Blutstrom einfließenden Infusionslösungen oder Medikamente können eine zeitlich verzögerte oder nur langsame Durchmischung des Blutes mit der zugegebenen Infusionslösung bedingen. Diese langsame Vermischung der beiden Flüssigkeiten an der Zugabestelle ist speziell bei der Zugabe der Calciumlösung zu Blut unerwünscht und kann aufgrund punktuell hoher Calciumkonzentrationen im Blut vereinzelt zur Gerinnselbildung flussabwärts führen.

Um dies zu verhindern, ist die rasche und homogene Durchmischung der zugegebenen Calciumlösung mit dem Blut wünschenswert.

Das Problem kann sich aber auch bei der Zumischung anderer Flüssigkeiten ergeben, z. B. Medikamenten, deren schnelle Durchmischung ebenfalls vorteilhaft sein kann.

Im Stand der Technik ist dieses Problem bekannt und zur Lösung werden spezielle Zugabestellen, die Mittel zur Turbulenzerzeugung enthalten, beschrieben.

In der WO 2014/026771 A1 wird ein Einsatzstück für ein Blutschlauchsystem mit einer Spiralstruktur beschrieben. Die Spiralstruktur dient dem Erzeugen von Turbulenzen im Bereich der Infusionsstelle. Die Turbulenzen sollen einer besseren Vermischung dienen.

In der DE 42 40 681 A1 ist eine Vorrichtung zur Hämodialyse ohne Antikoagulation offenbart.

Aus der EP 1 666 078 A2 sind eine extrakorporale Blutbehandlung und entsprechende Vorrichtungen bekannt.

Eine erfindungsgemäße Aufgabe kann darin bestehen, eine weitere Lösung zum Begünstigen einer Vermischung einer Infusionslösung mit einem weiteren Fluid, etwa Blut, vorzuschlagen.

Die Aufgabe kann gelöst werden durch eine Zugabeleitung mit den Merkmalen des Anspruchs 1, einen extrakorporalen Blutkreislauf mit den Merkmalen des Anspruchs 4 sowie eine Blutbehandlungsvorrichtung mit den Merkmalen des Anspruchs 6. Erfindungsgemäß wird somit eine Zugabeleitung vorgeschlagen, die zum Zugeben einer Infusionslösung zu einem Fluid, welches in einem extrakorporalen Blutkreislauf strömt, bestimmt ist. Die Zugabeleitung ist daher zu ihrer Verbindung mit einem extrakorporalen Blutkreislauf vorgesehen und optional bereits entsprechend ausgestaltet, beispielsweise durch entsprechende Verbinder, T-Stücke, oder dergleichen. Die erfindungsgemäße Zugabeleitung weist wenigstens ein Druckentlastungsventil auf, wobei das Druckentlastungsventil konfiguriert ist, um einen Durchfluss hierdurch erst dann zu erlauben, wenn sich ein ausreichend hoher Druck stromauf hiervon eingestellt hat, so dass, auch wenn die Infusionslösung mittels einer Pumpe kontinuierlich entlang der Zugabeleitung in Richtung des Druckentlastungsventils gefördert wird, mittels des Druckentlastungsventils ein pulsierender Strom an Infusionslösung erzeugt wird.

Der erfindungsgemäße extrakorporale Blutkreislauf weist wenigstens eine Blutrückgabeleitung und eine erfindungsgemäße Zugabeleitung auf.

Die erfindungsgemäße Blutbehandlungsvorrichtung ist mit einem erfindungsgemäßen extrakorporalen Blutkreislauf verbunden. Letzterer weist, wie vorstehend festgehalten, zumindest eine erfindungsgemäße Zugabeleitung und eine Blutrückgabeleitung auf. Die Blutbehandlungsvorrichtung weist ferner eine Infusionspumpe zum Fördern einer Infusionslösung innerhalb der Zugabeleitung oder durch diese hindurch auf, oder sie ist mit einer solchen Infusionspumpe körperlich oder in Signalverbindung verbunden. Ferner weist sie eine Blutpumpe zum Fördern von Blut innerhalb der Blutrückgabeleitung oder durch diese hindurch auf. Die Infusionspumpe wird mittels einer Steuervorrichtung oder Regelvorrichtung gesteuert (im Folgenden kurz: Steuervorrichtung) oder geregelt. Eine solche ist vorgesehen.

Das hierin offenbarte erfindungsgemäße Verfahren dient nicht dem Steuern einer Infusionspumpe zum Fördern einer Infusionslösung innerhalb einer erfindungsgemäßen Zugabeleitung. Es umfasst zumindest ein Bereitstellen einer erfindungsgemäßen Zugabeleitung, ein Verbinden der Infusionspumpe mit einer Quelle für die Infusionslösung, ein Verbinden oder Inkontaktbringen der Infusionspumpe mit einem Pumpabschnitt der Zugabeleitung, und ein Fördern der Infusionslösung durch, vorzugsweise kontinuierliches oder andauerndes, Betreiben der Infusionspumpe unter einer kontinuierlichen Förderrate.

Erfindungsgemäße Ausführungsformen können eines oder mehrere der vorstehend oder im Folgenden genannten Merkmale in beliebiger Kombination aufweisen.

Erfindungsgemäße Ausführungsformen sind ferner Gegenstand der Unteransprüche.

Bei allen hierin gemachten Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Hierin gemachte räumliche Angaben wie "oben", "unten", usw. beziehen sich im Zweifel auf die Darstellung, wie sie den hier beigefügten Figuren zu entnehmen ist.

Das Druckentlastungsventil kann ein Rückschlagventil sein.

In bestimmten, beispielhaften erfindungsgemäßen Ausführungsformen ist das Druckentlastungsventil in einem Abschnitt der Zugabeleitung angeordnet. Dieser Abschnitt liegt zwischen einem Pumpabschnitt der Zugabeleitung, welcher vorgesehen und/oder ausgestaltet ist, um in eine Infusionspumpe eingelegt oder mit dieser verbunden zu werden, und einem Verbindungsabschnitt, welcher vorgesehen und/oder ausgestaltet ist, um mit einer Blutleitung eines extrakorporalen Blutkreislaufs in Fluidkommunikation verbunden zu werden.

In bestimmten, beispielhaften erfindungsgemäßen Ausführungsformen weist die Zugabeleitung eine Quelle für die Infusionslösung auf oder ist hiermit in Fluidverbindung verbunden.

Die Infusionslösung kann eine Calciumlösung sein.

In bestimmten, beispielhaften erfindungsgemäßen Ausführungsformen ist die Steuervorrichtung eingerichtet oder programmiert, um die Infusionspumpe ausschließlich kontinuierlich fördern zu lassen.

In bestimmten, beispielhaften erfindungsgemäßen Ausführungsformen ist die Zugabeleitung in Fluidkommunikation mit der Blutentnahmeleitung verbunden.

In bestimmten, beispielhaften erfindungsgemäßen Ausführungsformen ist die Steuervorrichtung ausdrücklich nicht eingerichtet oder programmiert, um die Infusionspumpe pulsierend fördern zu lassen.

Eine pulsierende Fördertätigkeit oder ein sich hieraus ergebendes gepulstes Fluss- oder Förderprofil, welches erwünschtes oder gezieltes Ergebnis der Steuerung ist, welches also durch entsprechende Steuersignale erzeugt wird, ist in einigen beispielhaften erfindungsgemäßen Ausführungsformen ausdrücklich nicht Gegenstand oder Teil der vorliegenden Erfindung.

Bei einem gepulsten Flussprofil können sich Phasen, in denen die Flüssigkeit oder die Infusionslösung mittels der Infusionspumpe gefördert wird (Flusspulse oder Pulse) und Phasen, in denen keine oder deutlich weniger Flüssigkeit gefördert wird (Flusspausen), periodisch abwechseln. Dies führt zu einem sogenannten gepulsten Flussprofil. In anderen Worten, die Infusionspumpe läuft diskontinuierlich, was bedeutet, dass in der Förderrate der Infusionspumpe prägnante Änderungen auftreten. Die Infusionspumpe muss zum Erzeugen eines solchen gepulsten Flussprofils nicht unbedingt in periodischen Abständen oder regelmäßig gestoppt werden. Eine derartige Steuerung ist nicht Gegenstand der vorliegenden Erfindung.

Ein gepulstes Flussprofil kann dabei ein Rechteck-Profil aufweisen, ein sinusförmiges Profil, ein nadelförmiges Profil oder ähnliche.

In einer Ausführungsform des nicht erfindungsgemäßen Verfahrens kann die Steuereinrichtung die Infusionspumpe derart steuern oder regeln, dass diese kontinuierlich läuft, um die Infusionslösung mit einem stetigen Fluss zu fördern.

Unter einem kontinuierlichen Fördern kann ein nicht-gepulstes Fördern verstanden werden.

Für den Fachmann ist selbstverständlich, dass, bedingt durch die Struktur der Infusionspumpe, z. B. eine okkludierende Infusionspumpe, Druck- und Flusspulse entstehen können. Dieses von der Pumpenstruktur, nicht aber von einer Steuervorrichtung, technisch bedingte Flussprofil zählt in bestimmten erfindungsgemäßen, beispielhaften Ausführungsformen nicht als ein gepulstes Flussprofil im Sinne der vorliegenden Erfindung und darf daher erfindungsgemäß auftreten. Ein hingegen von einer Steuervorrichtung erzeugte gepulstes Flussprofil ist, wie oben ausgeführt, im Unterschied hierzu nicht von der vorliegenden Erfindung erfasst. Ein allein von der Pumpenstruktur ohne Mitwirkung des hierin beschriebenen Druckentlastungsventils erzeugtes gepulstes Flussprofil kann, da dies möglicherweise bereits im üblichen Gebrauch einer Infusionspumpe auftreten kann, selbst wenn die Infusionspumpe konstant betriebenen wird, hingegen auch erfindungsgemäß auftreten.

In bestimmten, beispielhaften erfindungsgemäßen Ausführungsformen erfolgt das Fördern solange mit einer kontinuierlichen Förderrate, wie die Blutpumpe mit einer kontinuierlichen Förderrate fördert, oder stets dann, wenn die Blutpumpe mit einer kontinuierlichen Förderrate fördert.

In bestimmten, beispielhaften erfindungsgemäßen Ausführungsformen erfolgt das Fördern solange mit einer kontinuierlichen ersten Förderrate, bis eine manuelle oder automatische Veränderung der Förderrate der Infusionspumpe vorgenommen wird. Anschließend kann erneut mit einer kontinuierlichen zweiten Förderrate gefördert werden, wobei sich die erste Förderrate von der zweiten unterscheiden kann.

Der erfindungsgemäße extrakorporale Blutkreislauf kann neben der blutführenden Hauptleitung eine oder mehrere Infusionsleitungen aufweisen, die über T-Stücke mit einer blutführenden Leitung verbunden sind. T-Stücke können Kunststoffteile mit drei Öffnungen sein, die über eine T-förmige Fluidleitung verbundenen sind. Die Fluidleitung kann auch y-förmig oder ähnlich ausgebildet sein. T-Stücke können als Einsatzstücke in ein Schlauchsystem eingeklebt werden, wodurch die Hauptleitung des Schlauchsystems, z. B. die blutführende Leitung, mit der Infusionsleitung verbunden werden kann.

Einige oder alle erfindungsgemäßen Ausführungsformen können einen oder mehrere der oben oder im Folgenden genannten Vorteile aufweisen.

Ein Vorteil besteht darin, dass durch das Einspritzen der Infusionslösung in das Blut - im Gegensatz zu einem kontinuierlichen Einbringen, wie dies im Stand der Technik üblich ist - eine kurzzeitige Verwirbelung der Infusionslösung innerhalb des Bluts erzeugt wird. Dies beschleunigt die Durchmischung der Infusionslösung mit dem Blut.

Ein Vorteil der Verwendung der erfindungsgemäßen Zugabeleitung oder des erfindungsgemäßen Blutkreislaufs kann darin bestehen, dass die hierin beschriebenen Vorteile erzielbar sein können, ohne dass hierzu eine Veränderung an der Steuerung der Blutbehandlungsvorrichtung oder der Pumpe für die Infusionslösung vorgenommen werden müsste. Denn die Verwendung der erfindungsgemäßen Zugabeleitung bewirkt keine Veränderung in der Menge oder der Konzentration der dem Patienten zugeführten Infusionslösung. Der erfindungsgemäße Effekt wird mit den bekannten Vorrichtungen allein durch die pulsierende Zugabe, bewirkt allein mittels der Auf-Zu-Funktion des Druckentlastungsventils, erzielt. Insbesondere fördert die Infusionspumpe für die Infusionslösung nicht anders als im Stand der Technik, nämlich vorzugsweise kontinuierlich. Ihr Förderverhalten kann beibehalten bleiben.

Damit einher geht vorteilhaft, dass es keiner baulichen oder andersartigen Anpassung bereits ausgelieferter Blutbehandlungsvorrichtungen, etwa einer Softwareanpassung, bedarf. Die Umsetzung der vorliegenden Erfindung kann aus dem Verwenden von erfindungsgemäßen Zugabeleitungen oder Blutschlauchsätzen bestehen. Ein Umstellen auf Letztere ist auch deshalb einfach zu bewerkstelligen, da es sich hierbei ohnehin um Einwegartikel oder Disposables handelt.

Die Wirkung der erfindungsgemäßen Zugabeleitung ist unabhängig von Parametern der Blutbehandlung. Wird beispielsweise die Förderrate der Blutpumpe und im Einklang hiermit die Förderrate der Pumpe für die Infusionslösung verändert, so ändert dies nichts am Wirkprinzip der erfindungsgemäßen Zugabeleitung. Allein die Frequenz, mit welcher das Druckentlastungsventil öffnet, kann sich - selbsttätig - an die veränderte(n) Förderrate(n) anpassen.

Die vorliegende Erfindung wird im Folgenden anhand der beigefügten Zeichnungen, in welcher identische Bezugszeichen gleiche oder ähnliche Bauteile bezeichnen, exemplarisch erläutert. In den zum Teil stark vereinfachten Figuren gilt:
- Fig. 1: zeigt die Anordnung einer erfindungsgemäßen Zugabeleitung an einem erfindungsgemäßen extrakorporalen Blutschlauchsatz;
- Fig. 2: zeigt Details der Zugabeleitung der Fig. 1 mit einem Druckentlastungsventil; und
- Fig. 3: zeigt den Eingangsdruck, der am Druckentlastungsventil der Fig. 2 anliegen kann, und die dem Eingangsdruck jeweils entsprechende Geschwindigkeit einer Infusionslösung beim Durchströmen der Zugabeleitung.

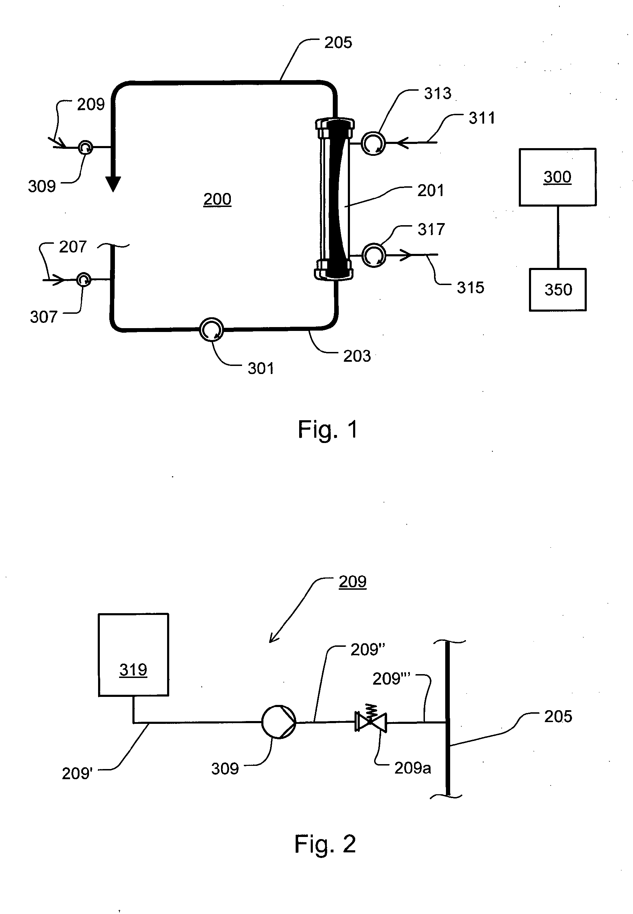

**Fig. 1** zeigt die grundsätzliche Anordnung einer erfindungsgemäßen Zugabeleitung 209 an einem schematisch sehr vereinfacht dargestellten, erfindungsgemäßen extrakorporalen Blutschlauchsatz 200 in einer exemplarischen Ausführungsform des Blutschlauchsatzes.

Der Blutschlauchsatz 200 weist einen Hämofilter 201 auf. Mit dem Hämofilter 201, oder Dialysator oder Blutfilter, sind eine Blutentnahmeleitung 203 (oder arterielle Leitung) und eine Blutrückgabeleitung 205 (oder venöse Leitung) verbunden.

Die Blutentnahmeleitung 203 ist mit einer Blutpumpe 301 in Wirkverbindung verbunden oder weist diese auf.

Stromauf der Blutpumpe 301 mündet eine weitere Zugabeleitung, hier eine Leitung 207 für Citratlösung, in die Blutentnahmeleitung 203.

Die Leitung 207 ist mit einer Citratpumpe 307 in Wirkverbindung verbunden oder weist diese auf.

Stromab des Hämofilters 201 mündet die erfindungsgemäße Leitung 209 für Calciumlösung in die Blutrückgabeleitung 205.

Die erfindungsgemäße Leitung 209 ist mit einer Calciumpumpe 309 in Wirkverbindung verbunden oder weist diese auf. Sie wird aus einer nur in Fig. 2 gezeigten Quelle für eine Infusionslösung, hier exemplarisch eine Calciumquelle 319, gespeist. Die Quelle kann ein Beutel oder eine Flasche sein. Optional kann die Infusionslösung on-line erzeugt sein; in diesem Fall zählt die entsprechende Vorrichtung, in welcher erzeugt wird, als Quelle.

Der Hämofilter 201 ist mit einer Leitung 311 für frische Dialysierflüssigkeit und einer Leitung 315 für verbrauchtes Dialysat oder Filtrat verbunden. Die Leitung 311 ist mit einer Dialysierflüssigkeitspumpe 313 verbunden oder weist diese auf. Die Leitung 315 ist mit einer Filtratpumpe 317 verbunden oder weist diese auf.

Die gezeigten Pfeilspitzen geben jeweils die Strömungsrichtung in einer bestimmungsgemäßen Benutzung des Blutschlauchsatzes 200 an.
Der in Fig. 1 gezeigt Blutschlauchsatz 200 kann einem üblichen extrakorporalen Blutschlauchsatz entsprechen und insbesondere für die CWHD (*continuous veno-venous hemodialysis*) geeignet sein, mit Ausnahme der in Fig. 2 detailliert gezeigten erfindungsgemäßen Leitung 209 für Calcium als Beispiel einer erfindungsgemäßen Zugabeleitung für Infusionslösungen.

Die Pumpen 301, 307, 309, 313 und 317 können Teil einer nur schematisch angedeuteten Blutbehandlungsvorrichtung 300 sein. Dasselbe gilt für die Leitungen 311 und 315.

Die Blutbehandlungsvorrichtung 300 kann eine Steuervorrichtung 350 aufweisen oder hiermit verbunden sein.

Die Steuervorrichtung 350 kann zum Steuern oder zum Regeln ausgestaltet und verwendet werden. Sie kann eine Regelvorrichtung sein.

Die Steuervorrichtung 350 kann Teil der Blutbehandlungsvorrichtung 300 sein, sie kann extern zur Blutbehandlungsvorrichtung 300 sein oder getrennt von dieser vorliegen. Die Steuervorrichtung kann programmiert sein, um die Calciumpumpe 309 - und optional auch weitere Pumpen, insbesondere die vorstehend genannten - oder andere Komponenten der Blutbehandlungsvorrichtung 300, zu steuern oder zu regeln.

**Fig. 2** zeigt schematisch wiederum sehr vereinfacht Details der Zugabeleitung bzw. Leitung 209 für Calcium der Fig. 1 mit einem Druckentlastungsventil 309 in einer beispielhaften erfindungsgemäßen Ausführungsform der Leitung 209 oder der Zugabeleitung.

Die Leitung 209 erstreckt sich zwischen der Quelle 319 für Infusionslösung, hier eine Calciumlösungsquelle, und einer Blutleitung, hier die Blutrückgabeleitung 205, also die venösen Patientenleitung.

Dabei steht die Leitung 209 in Wirkverbindung mit der Calciumpumpe 309 oder weist diese auf, wie zu Fig. 1 erläutert.

Stromab der Calciumpumpe 309, welche bei der Behandlung in Richtung Blutrückgabeleitung 205 fördert, weist die Leitung 209 ein Druckentlastungsventil 209a auf oder ist hiermit verbunden. Das Druckentlastungsventil 209a kann ein übliches Rückschlagventil sein.

Bedingt durch die Calciumpumpe 309 und das Druckentlastungsventil 209a lässt sich die Leitung 209 in Abschnitt 209', welcher sich zwischen der Quelle 319 und der Pumpe 309 erstreckt, Abschnitt 209", welcher sich zwischen der Pumpe 309 und dem Druckentlastungsventil 209a erstreckt, und Abschnitt 209"', welcher sich zwischen Druckentlastungsventil 209a und Verbindung mit der Blutrückgabeleitung 205 erstreckt, unterteilen.

Das Druckentlastungsventil 209a erlaubt einen Durchfluss hierdurch erst dann, wenn sich ein ausreichend hoher Druck stromauf hiervon eingestellt hat. Bei kontinuierlich fördernder Pumpe 309 wird mittels (ausschließlich) des Druckentlastungsventils 209a somit ein pulsierender Strom an Infusionslösung erzeugt, welcher in die Blutleitung einfließen kann.

Vorteilhaft ist es, wenn die Kopplung zwischen Pumpe 309 und Druckentlastungsventil 209a gedämpft ist. Dies kann beispielsweise dadurch bewirkt werden, dass das Schlauchmaterial der Leitung 209 zumindest in ihrem Abschnitt 209", also zwischen Pumpe 309 und Druckentlastungsventil 209a, eine elastische Verformung erlaubt oder elastisch verformbar ist. Abschnitt 209" kann daher beispielsweise als Kunststoffschlauch, nicht aber als Metallrohr ausgestaltet sein. Durch die elastische Verformbarkeit kann ein optionales Lösungsmittelreservoir zwischen Pumpe 309 und Druckentlastungsventil 209a geschaffen werden, welches jenes von der Pumpe 309 zwischen einem Schließen des Druckentlastungsventils 209a und dem erneuten Öffnen geförderte Fluidvolumen aufnehmen kann.

Zudem kann sich optional bei ausreichend langsam schließendem Druckentlastungsventil 209a, wie dies erfindungsgemäß ebenfalls vorgesehen sein kann, ein Druckabfall (messbar direkt stromauf des Druckentlastungsventils 209a) bis unter den Öffnungsdruck des Druckentlastungsventils 209a einstellen.

Unter einem ausreichend langsam schließenden Druckentlastungsventil kann in einigen erfindungsgemäßen Ausführungsformen ein Ventil verstanden werden, welches - optional im Zusammenspiel mit einem geeigneten Schlauch, etwa wie vorstehend beschrieben - ein pulsierendes Fördern ermöglicht. Ein Ventil, welches derart rasch auf Druckänderungen reagiert, dass ein Druck unmittelbar stromauf des Ventils nicht bis unter Öffnungsdruck abfallen kann, gilt nicht als ausreichend langsam im vorliegenden Sinne.

Als "ausreichend langsam" ist hingegen ein Druckausgleichventil zu verstehen, welches bei einem Öffnungsdruck öffnet und bei einem vorbestimmten oder bestimmbaren und vorzugsweise konstanten Schließdruck wieder schließt, wobei der Schließdruck niedriger ist als der Öffnungsdruck. Als "ausreichend langsam" ist ferner auch ein Druckausgleichventil zu verstehen, welches bei einem Öffnungsdruck zu einem ersten Zeitpunkt öffnet und erst nach Ablauf einer vorbestimmten oder ausreichenden Zeitdauer zu einem späteren, zweiten Zeitpunkt wieder schließt. Auch dies kann dazu führen, dass das Ventil bei einem Schließdruck schließt, welcher unter dem Öffnungsdruck liegt.

Die Größe oder das Volumen des vorstehend genannten Lösungsmittelreservoirs zwischen Pumpe 309 und Druckentlastungsventil 209a kann beispielsweise durch Auswählen einer konkreten Länge des Abschnitts 209" festgelegt werden. Ein erfindungsgemäß sinnvoller Wert beträgt 1 ml.

Die Pumpe 309 kann als Rollerpumpe, Kolbenpumpe, Zentrifugalpumpe, Membranpumpe oder auf jede andere Art ausgestaltet sein. Insbesondere kann sie peristaltisch oder okkludierend sein. Vorzugsweise aber ist die Pumpe 309 eine kontinuierlich fördernde Pumpe, welche tatsächlich auch kontinuierlich fördernd, d. h. mit konstanter Förderleistung, eingesetzt wird.

Die Förderleistung kann erfindungsgemäß auf Werte wie 0,1 ml/min, 0,6 ml/min, 1,4 ml/min, 2 ml/min und jeden beliebigen Zwischenwert eingestellt werden.

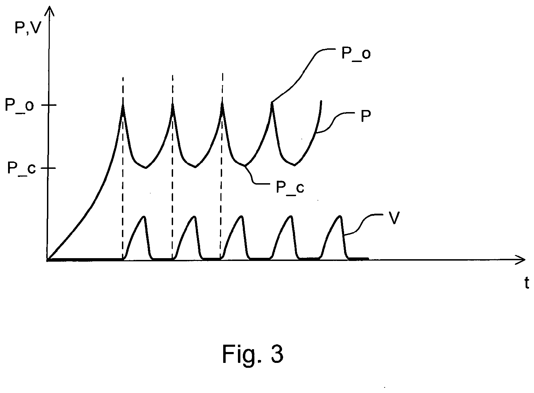

**Fig. 3** zeigt in einem gemeinsamen Diagramm einen Eingangsdruck p und eine Flussgeschwindigkeit v über der Zeit t.

Der Eingangsdruck p ist jener Druck, der zu einem Zeitpunkt t am Eingang des Druckentlastungsventils 309 der Fig. 2 anliegen kann, also insbesondere im Abschnitt 209" der Leitung 209.

Die Flussgeschwindigkeit v gibt die dem Eingangsdruck p jeweils entsprechende Geschwindigkeit einer Infusionslösung an, mit welcher diese die Leitung 209 und insbesondere deren Abschnitt 209'" durchströmt.

Im Diagramm der Fig. 3 sind der Öffnungsdruck p_o und der Schließdruck p_c für das Druckentlastungsventil 209a gezeigt. Beim Öffnungsdruck p_o ist der erforderliche Durck erreicht, bei welchem das Druckentlastungsventil 209a mit dem Öffnen beginnt. Beim Schließdruck p_c ist das Druckentlastungsventil 209a vollständig geschlossen.

Die in Fig. 3 gezeigten Verläufe sind als idealisiert zu betrachten.

### Bezugszeichenliste

- 200: Blutschlauchsatz
- 201: Hämofilter oder Blutfilter oder Dialysator
- 203: Blutentnahmeleitung
- 205: Blutrückgabeleitung
- 207: Leitung für Citratlösung
- 209: Leitung für Calciumlösung; Zugabeleitung
- 209': Abschnitt
- 209": Abschnitt
- 209'": Abschnitt
- 209a: Druckentlastungsventil

- 300: Blutbehandlungsvorrichtung
- 301: Blutpumpe
- 307: Citratpumpe
- 309: Calciumpumpe
- 311: Leitung für Dialysierflüssigkeit
- 313: Pumpe für Dialysierflüssigkeit
- 315: Leitung für Dialysat, Filtrat
- 317: Pumpe für Dialysat, Filtrat
- 319: Quelle für Infusionslösung, Calciumquelle
- 350: Steuer- oder Regelvorrichtung

## Patentansprüche

1. Zugabeleitung (209) zum Zugeben einer Infusionslösung zu einem Fluid, welches in einem extrakorporalen Blutkreislauf (200) strömt, wobei die Zugabeleitung (209) ein Druckentlastungsventil (209a) oder ein Rückschlagventil aufweist, **dadurch gekennzeichnet, dass** das Druckentlastungsventil (209a) konfiguriert ist, um einen Durchfluss hierdurch erst dann zu erlauben, wenn sich ein ausreichend hoher Druck stromauf hiervon eingestellt hat, so dass, auch wenn die Infusionslösung mittels einer Pumpe (309) kontinuierlich entlang der Zugabeleitung (209) in Richtung des Druckentlastungsventils (209a) gefördert wird, mittels des Druckentlastungsventils (209a) ein pulsierender Strom an Infusionslösung erzeugt wird.

2. Zugabeleitung (209) nach Anspruch 1, welche eine Quelle (319) für die Infusionslösung aufweist oder hiermit in Fluidverbindung verbunden ist.

3. Zugabeleitung (209) nach Anspruch 1 oder 2, wobei die Infusionslösung eine Calciumlösung ist oder aufweist.

4. Extrakorporaler Blutkreislauf (200), welcher wenigstens eine Blutrückgabeleitung (205) und eine Zugabeleitung (209) nach einem der Ansprüche 1 bis 3 aufweist.

5. Extrakorporaler Blutkreislauf (200) nach Anspruch 4, wobei die Zugabeleitung (209) in Fluidkommunikation mit der Blutrückgabeleitung (205) verbunden ist.

6. Blutbehandlungsvorrichtung (300), welche verbunden ist mit einem extrakorporalen Blutkreislauf (200) gemäß Anspruch 4 oder 5 mit einer Zugabeleitung (209) und einer Blutrückgabeleitung (205), wobei die Blutbehandlungsvorrichtung (300) eine Infusionspumpe (309) zum Fördern einer Infusionslösung innerhalb der Zugabeleitung (209) und eine Blutpumpe (301) zum Fördern von Blut innerhalb der Blutrückgabeleitung (205) aufweist, wobei die erste Pumpe (309) mittels einer Steuervorrichtung (350) gesteuert wird.

7. Blutbehandlungsvorrichtung (300) nach Anspruch 6, wobei die Steuervorrichtung (350) eingerichtet oder programmiert ist, um die Infusionspumpe (309) ausschließlich kontinuierlich fördern zu lassen.

8. Blutbehandlungsvorrichtung (300) nach Anspruch 6 oder 7, wobei die Steuervorrichtung (350) nicht eingerichtet oder programmiert ist, um die Infusionspumpe (319) pulsierend fördern zu lassen.

## Claims

1. A feed line (209) for adding an infusion solution to a fluid flowing in an extracorporeal blood circuit (200), the feed line (209) comprising a pressure relief valve (209a) or a non-return valve,
**characterized in that** the pressure relief valve (209a) is configured to allow a flow therethrough only when a sufficiently high pressure has been set upstream thereof, so that, even when the infusion solution is continuously conveyed by means of a pump (309) along the feed line (209) in the direction of the pressure relief valve (209a), a pulsating stream of infusion solution is generated by means of the pressure relief valve (209a).

2. The feed line (209) according to claim 1, comprising a source (319) for the infusion solution or being connected in fluid communication therewith.

3. The feed line (209) according to claim 1 or 2, wherein the infusion solution is or comprises a calcium solution.

4. An extracorporeal blood circuit (200), comprising at least one blood return line (205) and a feed line (209) according to anyone of claims 1 to 3.

5. The extracorporeal blood circuit (200) according to claim 4, wherein the feed line (209) is connected in fluid communication with the blood return line (205).

6. A blood treatment apparatus (300) which is connected to an extracorporeal blood circuit (200) according to claim 4 or 5 comprising a feed line (209) and a blood return line (205), wherein the blood treatment apparatus (300) comprises an infusion pump (309) for conveying an infusion solution within the feed line (209) and a blood pump (301) for conveying blood within the blood return line (205), the first pump (309) being controlled by means of a control apparatus (350).

7. The blood treatment apparatus (300) according to claim 6, wherein the control apparatus (350) is set up or programmed, to allow the infusion pump (309) to convey exclusively continuously.

8. The blood treatment apparatus (300) according to claim 6 or 7, wherein the control apparatus (350) is not set up or programmed to allow the infusion pump (319) to convey pulsatively.

## Revendications

1. Une conduite d'addition (209) permettant d'ajouter une solution pour perfusion à un fluide s'écoulant dans un circuit sanguin extracorporel (200), la conduite d'addition (209) comprenant une vanne de surpression (209a) ou une vanne anti-retour,
**caractérisée en ce que** la vanne de surpression (209a) est configurée de façon à permettre un passage au travers de celle-ci uniquement lorsqu'une pression suffisamment élevée s'est établie en amont de celle-ci, de sorte que, même si la solution pour perfusion est acheminée de manière continue au moyen d'une pompe (309) le long de la conduite d'addition (209) en direction de la vanne de surpression (209a), un courant pulsé de solution pour perfusion soit généré au moyen de la vanne de surpression (209a).

2. La conduite d'addition (209) selon la première revendication, comprenant une source (319) pour la solution pour perfusion ou étant reliée en communication fluidique avec celle-ci.

3. La conduite d'addition (209) selon la revendication 1 ou 2, où la solution pour perfusion est ou contient une solution de calcium.

4. Un circuit sanguin extracorporel (200), comprenant au moins une conduite de retour de sang (205) ainsi qu'une conduite d'addition (209) selon l'une quelconque des revendications 1 à 3.

5. Le circuit sanguin extracorporel (200) selon la revendication 4, où la conduite d'addition (209) est reliée en communication fluidique avec la conduite de retour de sang (205).

6. Un appareil de traitement du sang (300), relié à un circuit sanguin extracorporel (200) selon la revendication 4 ou 5, comprenant une conduite d'addition (209) ainsi qu'une conduite de retour de sang (205), où l'appareil de traitement du sang (300) comprend une pompe à perfusion (309) pour acheminer une solution pour perfusion à l'intérieur du conduit d'addition (209) ainsi qu'une pompe à sang (301) pour acheminer le sang à l'intérieur du conduit de retour de sang (205), la première pompe (309) étant commandée au moyen d'un appareil de commande (350).

7. L'appareil de traitement du sang (300) selon la revendication 6, où l'appareil de commande (350) est mis en place ou programmé pour laisser la pompe à perfusion (309) acheminer exclusivement en continu.

8. L'appareil de traitement du sang (300) selon la revendication 6 ou 7, où l'appareil de commande (350) n'est pas mis en en place ou programmé pour laisser la pompe à perfusion (319) acheminer par pulsation.
